# EUROPEAN PATENT APPLICATION

(11) **EP 2 564 775 A1**
(43) Date of publication of application: **06.03.2013**
(21) Application number: 12181786.0
(22) Date of filing: 24.08.2012
(51) Int. Cl.: A61B 5/1455

(54) **Method for controlling sensor placement time, physiological measurement apparatus, and sensor and computer program product for a physiological measurement apparatus**

(30) Priority: 31.08.2011 US 201113221971
(71) Applicant: General Electric Company, Schenectady, NY 12345 (US)
(72) Inventor: Sivonen, Tarja, 02610 Espoo (FI)
(74) Representative: Illingworth-Law, William Illingworth

(57) **Abstract**

A method for controlling sensor placement time, a physiological measurement apparatus and a sensor and computer program product for a physiological measurement apparatus are disclosed. In order to prevent skin injuries, occurrence of at least one predetermined event is monitored (201, 202), wherein the at least one predetermined event comprises at least one predetermined start event. Upon detection of any of the at least one predetermined start event, a respective timer is started (203), thereby to start at least one timer, wherein each timer is provided with dedicated timer settings. Upon expiration of any of the at least one timer, a notification indicative of a need to change sensor placement is generated (205).

## Description

### BACKGROUND OF THE INVENTION

This disclosure relates to physiological sensors that are, during a measurement process, in contact with human skin and tend to exert pressure on the skin. This disclosure also relates to physiological measurement devices that utilize such sensors or probes. Below, "sensor" is used to refer to such sensors and probes. A typical example of device/sensor combination is a pulse oximeter provided with a finger sensor.

Plethysmography refers to measurement of changes in the sizes and volumes of organs and extremities by measuring changes in blood volume. Photoplethysmography relates to the use of optical signals transmitted through or reflected by blood for monitoring a physiological parameter/variable of a subject. Conventional pulse oximeters use red and infrared photoplethysmographic (PPG) waveforms, i.e. waveforms measured respectively at red and infrared wavelengths, to determine oxygen saturation of pulsatile arterial blood of a subject. The two wavelengths used in a conventional pulse oximeter are typically around 660 nm (red wavelength) and 940 nm (infrared wavelength).

Pulse oximetry is at present the standard of care for continuous monitoring of arterial oxygen saturation (SpO₂). Pulse oximeters provide instantaneous in-vivo measurements of arterial oxygenation, and thereby an early warning of arterial hypoxemia, for example. Pulse oximeters also display the photoplethysmographic waveform, which can be related to tissue blood volume and blood flow, i.e. the blood circulation, at the site of the measurement, typically in finger or ear.

Traditionally, pulse oximeters use the above-mentioned two wavelengths, red and infrared, to determine oxygen saturation. Other parameters that may be determined in a two-wavelength pulse oximeter include pulse rate and peripheral perfusion index (PI), for example. Increasing the number of wavelengths to at least four allows the measurement of total hemoglobin (THb, grams per liter) and different hemoglobin types, such as oxyhemoglobin (HbO₂), deoxyhemoglobin (RHb), carboxyhemoglobin (HbCO), and methemoglobin (MetHb). In practice, a pulse oximeter designed to measure all hemoglobin species may be provided with 4 to 8 wavelengths (i.e. light sources) ranging from around 600 nm up to around 1000 nm.

One drawback related to pulse oximeter measurements is that the use of the sensor may cause skin and tissue damages at the measurement site, typically foot or hand/finger, especially during lengthy measurement sessions. These injuries are manifested in the form of blisters or burns on skin surface. Finger injury, for example, may be caused by one or more mechanisms, which may involve electrical, mechanical and/or chemical interaction with patient's skin tissue. A major reason for this type of skin injury is the mechanical pressure that the sensor exerts on the finger. However, the injury may also result from a low leakage current caused by a damaged sensor and/or from a topical reaction to the sensor material or to the residual manufacturing chemicals. A further reason that may cause an injury is the over-heating of the light emitting diodes of the sensor. Patient's age and state typically plays a significant role in the emergence of the injuries. For example, decreased blood flow may make the tissue more vulnerable to damages. Lowered blood flow may be caused by many reasons, such as age and medication, and prolonged pressure exerted by the sensor may also deteriorate peripheral blood circulation. The time needed for an injury to develop varies a lot depending on the sensor, sensor placement and/or patient's personal attributes. However, lengthy measurement sessions involve an increased risk of skin injury. When the patient is awake and well-oriented, (s)he is usually able to tell the nurse that the sensor is pinching or otherwise painful. However, a non-conscious patient has not the possibility to speak up when the sensor starts to hurt.

Even though the sensors are carefully designed in every aspect (mechanical, electrical, etc.) to eliminate the emergence of skin injuries at the measurement site, skin injuries still occur. Therefore, manufacturers of pulse oximeter sensors typically recommend that the placement of the sensor should be changed every 3 to 4 hours and that the sensor should not be placed too tightly. However, in practice the sensor placement is usually not reported during healthcare personnel shift reports (neonatal units may be exceptions). Furthermore, in intensive care units (ICUs) the healthcare personnel have so many parameters to follow up that the SpO₂ measurement site is usually changed only when the signal has deteriorated so much that there is an alarm about low saturation, poor signal, or noise. The patient might at that point already have a pressure burn at the measurement site.

Mechanisms have been developed which generate a user notification indicating that the measurement site of the pulse oximeter sensor should be changed. These mechanisms are based on evaluation of the plethysmographic data to determine if one or more characteristics of the signal data exhibit a trend indicating deteriorating signal quality due to measurement site degradation. However, these mechanisms evaluate the need to change the measurement site only in terms of the actual measurement, to keep the quality of the measurement high enough over an extended period of time. In terms of the skin injuries, a drawback related to these mechanisms is that the signal may remain normal even though skin/tissue damages start to develop. That is, an analysis of the quality or characteristics of the signal data does not necessarily reveal alterations in patient's skin, and therefore cannot efficiently prevent skin injuries from occurring.

### BRIEF DESCRIPTION OF THE INVENTION

The above-mentioned problems are addressed herein which will be comprehended from the following specification.

Occurrences of one or more predetermined events are monitored and upon detection of a predetermined start event at least one timer is started, which is provided with timer settings that depend on the detected event. Timer settings, which define the operation of the timer, may include one or more parameters that determine the length of the timer period and possible also rules that indicate when the timer is allowed to advance and when the advancing is restricted. For example, one event may trigger a timer that advances constantly (no advancing restrictions) and another event may trigger a timer that is allowed to advance only during intervals that fulfill a given criterion. As the expiration of a timer triggers a site change notification, the above use of one or more timers ensures that there will always be no more than a certain maximum time interval to the site change notification. Further, the length of the time interval may be dimensioned and controlled according to the risk of skin injury of the particular patient. For example, the length of the time interval may be shortened automatically if an event is detected that indicates increased risk of skin injury.

In an embodiment, a method for controlling sensor placement time in a physiological measurement apparatus comprises monitoring for occurrence of at least one predetermined event, wherein the at least one predetermined event comprises at least one predetermined start event and starting, upon detection of any of the at least one predetermined start event, a respective timer, thereby to start at least one timer, wherein each timer is provided with dedicated timer settings. The method further comprises generating, upon expiration of any of the at least one timer, a notification indicative of a need to change sensor placement.

In another embodiment, a physiological monitoring apparatus comprises a sensor attachable to a subject so that the sensor comes into contact with the subject's skin and an event detection unit configured to monitor for occurrence of at least one predetermined event, wherein the at least one predetermined event comprises at least one predetermined start event. The apparatus further comprises a timer control unit configured to start a respective timer in response to detection of any of the at least one a predetermined start event, thereby to start at least one timer, wherein each timer is provided with dedicated timer settings, and an indication unit configured to generate, in response to expiration of any of the at least one timer, a notification indicative of a need to change sensor placement.

In yet another embodiment, a sensor for a physiological monitoring apparatus comprises an event detection unit configured to monitor for occurrence of at least one predetermined event, wherein the at least one predetermined event comprises at least one predetermined start event and a timer control unit configured to start a respective timer in response to detection of any of the at least one a predetermined start event, thereby to start at least one timer, wherein each timer is provided with dedicated timer settings. The sensor further comprises an indication unit configured to generate, in response to expiration of any of the at least one timer, a notification indicative of a need to change sensor placement.

In a still further embodiment, a computer program product for a physiological monitoring device comprises a first program product portion configured to monitor for occurrence of at least one predetermined event, wherein the at least one predetermined event comprises at least one predetermined start event and a second program product portion configured to start a timer in response to detection of any of the at least one a predetermined start event, thereby to start at least one timer, wherein each timer is provided with dedicated timer settings. The computer program product further comprises a third program product portion configured to generate, in response to expiration of any of the at least one timer, a notification indicative of a need to change sensor placement.

Various other features, objects, and advantages of the invention will be made apparent to those skilled in the art from the following detailed description and accompanying drawings.

### BRIEF DESCRIPTION OF THE DRAWINGS

FIG. 1 is a block diagram illustrating one embodiment of a sensor and monitor system provided with sensor placement timing;
FIG. 2 is a flow diagram illustrating an example of the operation of the control and processing unit in terms of sensor placement timing;
FIG. 3 illustrates another embodiment of a sensor and monitor system provided with sensor placement timing;
FIG. 4 illustrates another example of the control and processing unit in terms of sensor placement timing;
FIG. 5 illustrates a further example of the operation of the control and processing unit in terms of sensor placement timing;
FIG. 6 illustrates a further embodiment of a sensor and monitor system provided with sensor placement timing; and
FIG. 7 illustrates an example of the operational entities of the control and processing unit of a monitor unit.

### DETAILED DESCRIPTION OF THE INVENTION

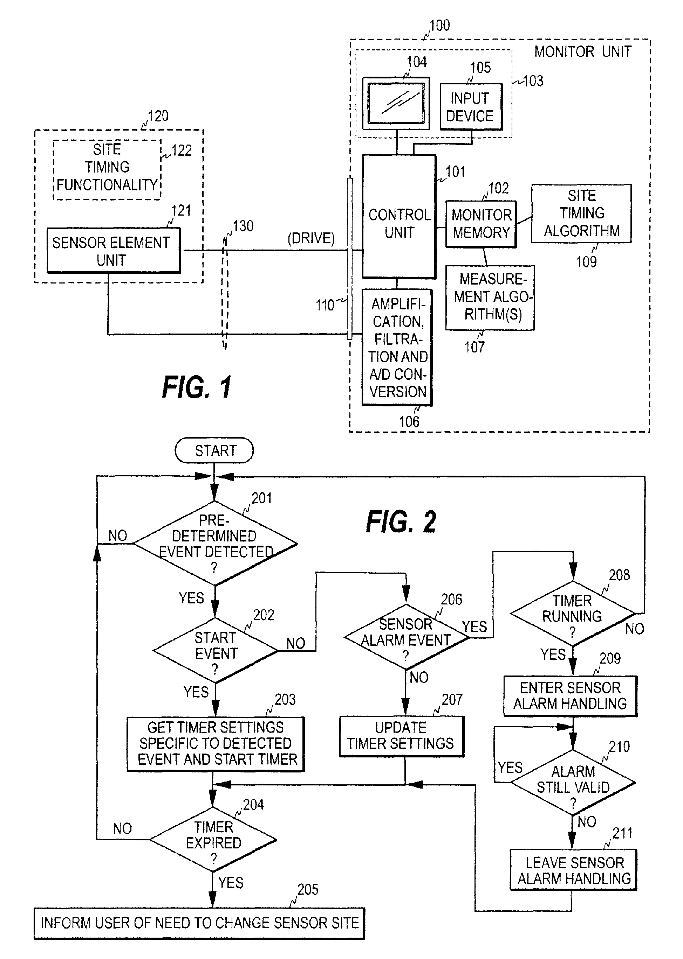

FIG. 1 illustrates one embodiment of a sensor and monitor system provided with site timing functionality. The sensor system of FIG. 1 comprises a monitor unit 100 and a sensor unit 120 attachable to a subject (not shown). The sensor unit 120 is normally connected through a cable 130 and a monitor interface 110 to the monitor unit, but the connection between the sensor unit and the monitor unit may also be wireless. It is to be noted that the system is here discussed with respect to one monitor unit 100 and one sensor unit 120 connected to the monitor unit. However, the entire system typically includes several sensor units 120 and one or more monitor units 100.

The monitor unit 100 may be conceived to comprise three basic elements: a computerized control and processing unit 101, a memory 102 for the control and processing unit, and a user interface 103, which typically comprises a display 104 and one or more user input devices 105.

A reception branch 106 of the monitor unit is adapted to receive the electrophysiological signals from the sensor. The reception branch typically comprises an input amplifier, a band-pass filter, and an A/D converter (not shown). The digitized signal output from the A/D converter is supplied to the control and processing unit 101, which processes the signal data and displays the analysis results on the screen of the display. The memory of the control and processing unit holds the measurement algorithm(s) 107 needed to process the data received from the sensor unit.

The sensor unit of FIG. 1 comprises a sensor element unit 121. In case of a pulse oximeter, the sensor element unit includes at least one light source for sending optical signals through the tissue and at least one photo detector for receiving the signal transmitted through or reflected from the tissue.

For limiting the amount of time the sensor is kept at a particular measurement site, the monitor unit may further be provided with a site timing algorithm 109 configured to inform the user when the sensor should be moved to another measurement site. Even though the site timing functionality is typically in the monitor unit, it may also reside in the sensor unit. This is illustrated with a dashed box denoted with reference numeral 122. The site timing functionality may also be distributed between the two units. It is assumed next that the functionality is implemented by algorithm 109 which is executable by the control and processing unit 101.

FIG. 2 illustrates an embodiment of the operation of the site timing algorithm. The algorithm is configured to detect predetermined events that may include start events and control events. A start event starts a timer, whereas a control event is used to control the operation/settings of a timer. The cessation of a "sensor off" message is a typical example of a start event, whereas a user action intended to adjust the settings of a timer, such as the period of the timer, is a typical control event. The "sensor off" message is commonly used in pulse oximeters and it may be generated based on comparison of the red and infrared signals. In connection with the start of a measurement session, the cessation of the message thus indicates that the sensor is now attached to the subject and data is received in a normal manner. As discussed below, the appearance of the "sensor off" message may be regarded as a control event if the message is received in the middle of a measurement session. In this case the message indicates that the sensor may not anymore be properly fitted and should therefore be checked.

The settings of each timer (not shown in FIG. 1) may be stored in the memory prior to the actual measurements. In response to the detection of a predetermined event at step 201, the site timing algorithm examines whether the event is a start event or a control event. In case of a start event, the site timing algorithm retrieves the timer settings associated with the detected start event and starts time measurement (steps 202/yes and 203) to measure the time elapsed from the start event. The process then constantly monitors for the expiration of the timer (step 204). If the predetermined event detected is not a start event but a control event (step 202/no), the process further checks if the event is a sensor alarm event or a regular control event (step 206). In case of a regular control event, the site timing algorithm retrieves the timer settings associated with the detected control event and updates the settings (step 207). If the respective timer is currently running, the update immediately effects the operation of the timer. If the timer is not running, the changes take effect next time the timer is started.

Further, the process constantly monitors if a new predetermined event is detected and starts or controls a timer depending on whether a start or control event is detected. When any of the started timers expires, the site timing algorithm informs the user that the measurement site of the sensor needs to be changed, cf. steps 204 and 205. The content of the user notification displayed in step 205 may depend on the timer expired, i.e. the process may also indicate the reason for the sensor placement change.

If it is detected in step 206 that the control event is a sensor alarm event, such as reception of the "sensor off" message, the process enters a sensor alarm handling mode (step 209) in which the user may be informed of the need to check the sensor and/or the fitting thereof. However, sensor alarm events are in this embodiment handled during the measurement only and therefore the process first checks at step 208, whether the measurement has already started, i.e. whether a timer is already running. If this is the case, the process monitors at step 210 for the cessation of the sensor alarm, which occurs when the user has corrected the fitting of the sensor. In response to the cessation of the sensor alarm, the process leaves the sensor alarm handling mode (step 211) and jumps to step 204 to continue the measurement.

In one embodiment, the predetermined events may comprise one start event only. In this embodiment, steps 201 and 202 may be combined and steps 206 to 210 omitted. Further, the arrow from step 204/no goes to the input of step 204. In this embodiment, the site timing algorithm thus starts, upon detection of a predetermined start event, a timer with a predetermined time interval, such as 4 hours, and displays a user notification when a predetermined time interval expires. This embodiment may further include a feature that allows the user to control the settings/operation of the timer at any stage of a measurement session. Further, the detection of sensor alarms that require a user action may affect the operation of the timer(s), and therefore a "run-time" sensor alarm handling mode is presented in FIG. 2. However, in some embodiments, the detection of sensor alarms, such as "sensor off" messages, may have no effect on the operation of a running timer. That is, a running timer may continue running regardless of a "sensor off" message.

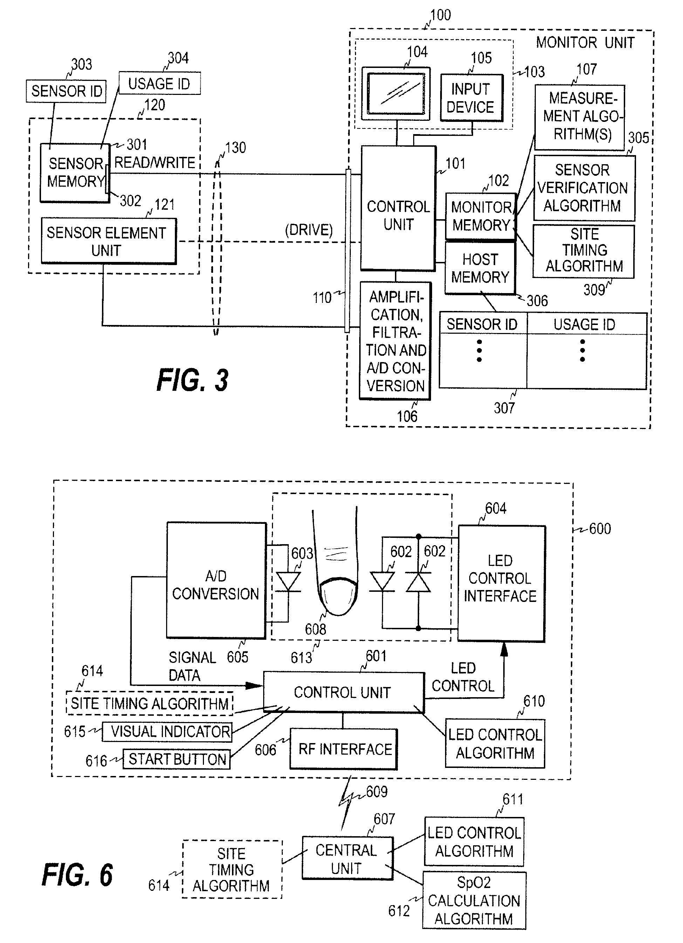

FIG. 3 illustrates another embodiment of the sensor and monitor system. This embodiment corresponds otherwise to the embodiment of FIG. 1, but is augmented with a verification mechanism configured to verify the authenticity of a sensor unit connected to the monitor unit. In FIG. 3, like reference numbers are used to denote similar elements as in FIG. 1 and below only the additional elements are described. Furthermore, the reference number of the site timing algorithm is now 309, since the operation of algorithm deviates from that of algorithm 109. In this example, the sensor unit also comprises a sensor memory 301. The sensor memory may be a generic memory from which the monitor unit may read data and into which the monitor unit may write data through a memory access interface 302. The sensor memory may thus be a plain (non-volatile) memory with no customized areas/parts, associated intelligence, or data processing capability. The memory may be, for example, an EPROM, EEPROM, or a flash memory. The memory holds a sensor-specific identifier 303 that unambiguously identifies the sensor and a sensor-specific usage identifier 304 that is indicative of the cumulative usage of the sensor. The sensor-specific identifier may be, for example, the serial number of the sensor. The usage identifier may be, for example, a usage count that indicates the total number of times the sensor unit has been used. The unique sensor-specific identifier may be stored at the manufacture stage of the memory or the sensor, and the usage identifier may be set to an initial value of zero at the manufacture stage of the sensor.

The memory 102 of the control and processing unit further holds a sensor verification algorithm 305 that is executed by the control and processing unit when a sensor is connected to the monitor unit 100. The operation of the verification algorithm is discussed below as if no encryption or any other data security mechanisms were involved. However, it is to be noted that the sensor memory data may be in encrypted form and various known data security mechanisms may be used to encrypt/decrypt the sensor memory data and/or to verify the authenticity of the sensor and/or the integrity of the sensor memory data. The sensor verification algorithm 305 may therefore include various data security mechanisms, in addition to the basic verification mechanism applied to plain data, i.e. non-encrypted data.

The monitor unit 100 is further provided with a host memory 306 which is here presented as a separate memory unit but which may also be a memory area of the monitor memory 102. The host memory contains the sensor-specific identifiers and sensor-specific usage identifiers for all sensors that have been used together with the monitor unit, i.e. that have been authenticated successfully by the monitor unit. This information may be in the form of a look-up table 307, for example. However, the look-up table or the host memory may also include further sensor-specific information needed by the sensor verification algorithm, such as the security parameters related to the possible data security mechanisms involved. For each authorized sensor the system thus includes two usage identifiers, one in the sensor and the other outside the sensor in a memory accessible by the monitor unit(s). As discussed below, inconsistency between the two usage identifiers is indicative of an unauthorized sensor. The system may also create the usage identifier pair in connection with the first use of a sensor, if both the sensor memory data and the host memory data indicate that the sensor has not been used before. The two usage identifiers of an authorized sensor may be unequal even though they are consistent with each other. That is, unequal usage identifiers are not necessarily inconsistent, although equal usage identifiers of a sensor are always consistent. Rather, inconsistency is in this embodiment detected if the usage count in the sensor unit is smaller than the usage count in the monitor unit, since it indicates that the sensor is likely an illegal copy.

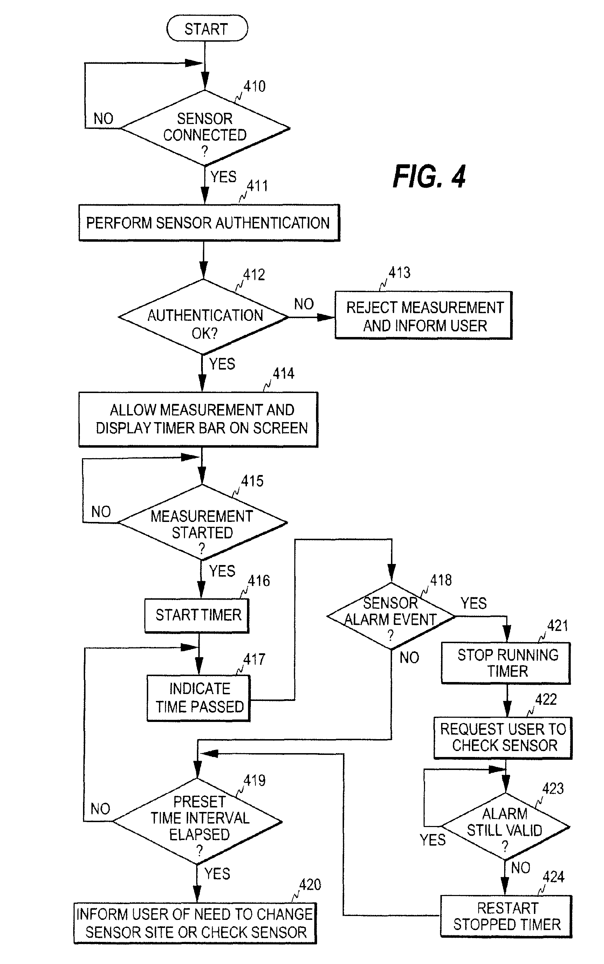

FIG. 4 illustrates an example of the operation of the control and processing unit 102 of FIG. 3 in terms of sensor placement timing. The control and processing unit monitors if a new sensor is connected to the monitoring unit (step 410). When a new sensor is detected, the control and processing unit starts an authentication process of the sensor at step 411. If the two usage identifiers are found to be consistent for the sensor identifier in question (step 412), the measurement is allowed and a timer bar is displayed on the screen of the display unit 104 (step 414). The timer bar may be green, for example, and the height of the bar may correspond to a preset change interval of the measurement site, such as 4 hours. The process then monitors when the actual measurement is started (step 415). Upon detection of the start of the measurement, a timer is started that also starts to update the timer bar shown on the screen (steps 416 and 417). The timer bar may continuously indicate the time passed and/or the time left before change of measurement site will be due.

When it is detected in step 419 that the preset time interval has elapsed, the user is informed of the need to change the sensor to another measurement location (step 420). If a sensor alarm event, such as a "sensor off" message, is detected (step 418/yes) before the timer expires, the timer may be stopped (step 421). A user notification may then be displayed to request the user to check the fitting of the sensor (step 422). When the user has corrected the fitting of the sensor, the alarm is turned off and the "sensor off" message disappears (step 423/yes). The process then restarts the timer (step 424) and continues to monitor for the expiration of the timer (step 419). Thus, in the embodiment of FIG. 4 the sensor alarm handling mode of FIG. 2 involves that the timer is paused for the period during which the sensor is not properly fitted at the measurement site.

If the two usage identifiers are found to be inconsistent in step 412, the measurement is rejected and the user is informed of the situation (step 413). The information displayed to the user may include a request to change the sensor.

In the embodiment of FIG. 4, the site timing functionality is provided with two trigger events; first an "authentication ok" signal obtained from step 412/yes, which triggers the retrieval of the timer settings and the display of the timer bar on the screen and then the actual start event, i.e. the start of the actual measurement, that starts the timer. Consequently, a start event may comprise a sequence of predetermined occurrences, the start event being detected upon completion of the sequence. A particular event may also trigger different operations depending on the time of the event. For example, the cessation of the "sensor off" message may be regarded as a start event (or as a part of a start event) that starts a timer period at the beginning of a measurement session, while the detection of the cessation may restart a paused timer in the middle of the measurement session.

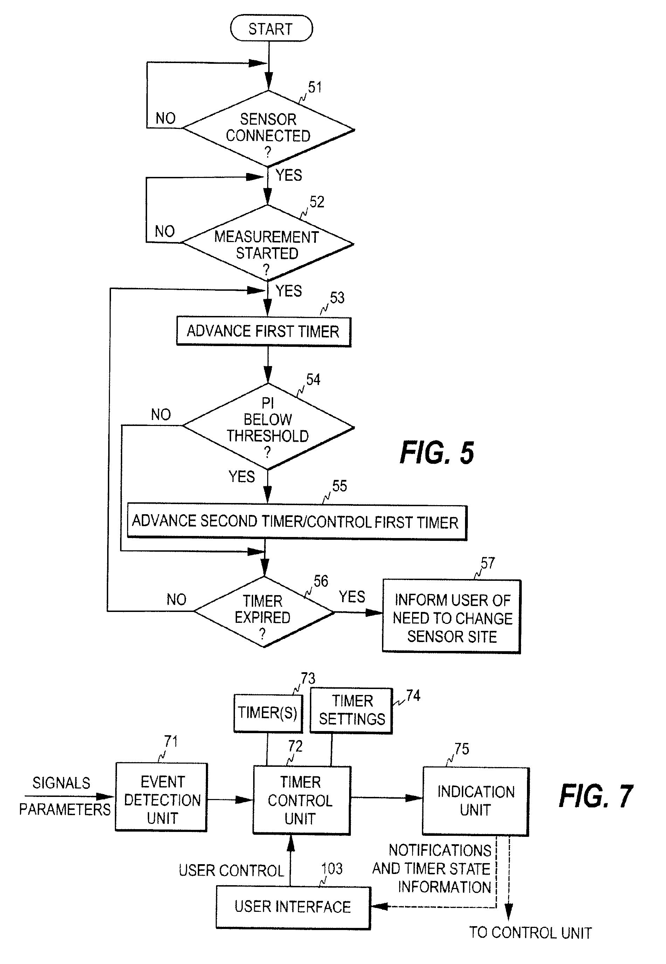

FIG. 5 illustrates another example of the operation of the site timing algorithm. It is assumed here that a pulse oximeter sensor is connected to the monitor unit. The control and processing unit first monitors if a new sensor is connected to the monitoring unit (step 51). When a new pulse oximeter sensor is detected, the control and processing unit further monitors when the actual measurement starts, i.e. when reception of plethysmographic data from the subject starts (step 52). Upon start of the measurement, the control and processing unit creates and starts a first timer (step 53). The first timer may be a timer that is advanced continuously for a preset period, such as 4 hours. The process then monitors the plethysmographic waveform and determines perfusion index (PI), which is a relative assessment of the pulse strength at the measurement site. As PI is primarily affected by the amount of blood flow at the measurement site, lowered PI is indicative of an increased risk of skin injury. The process therefore starts to monitor at step 54 whether PI remains above a given threshold value, which may be defined as a certain percentage of the original patient-specific value, for example. If it is detected at step 54 that PI drops below the threshold, the process may create and start a second timer (step 55). The second timer may advance only when PI remains below the threshold, thereby to track the total time that PI has remained under the threshold. Further, the period set for the second timer may depend on the time elapsed in the first timer. For example, the period of the second timer may be set to a period corresponding to a predefined proportion of the time period left in the first timer. When either of the two timers expires, the process indicates to the user that the location of the sensor should be changed (step 56). The reason for the location check may also be indicated.

In FIG. 5, the handling of sensor alarm events is not shown. However, sensor alarm events may be detected and processed similarly as in the embodiments of FIGS. 2 and 4.

Instead of starting and advancing a second timer, the process may also advance the first timer at a higher rate in step 55. That is, the drop of PI below the threshold may be regarded as a start event that starts a new timer or as a control event that controls an existing timer. Generally, it is not necessary to generate multiple timers but different events may be used as (predetermined) control events that control a single timer depending on the skin injury risk associated with the event. In addition to, or instead of, monitoring PI, the process may also monitor sensor motion, since motion of the sensor may also increase the risk of a skin injury. Therefore, a separate motion timer may advance when motion is detected in the plethysmographic signal data or the process may advance the first timer at a higher rate when motion is detected. A further parameter indicative of changes in the skin injury risk is a temperature value indicative of the temperature of skin surface at the measurement site. Thus, one or more parameters that correlate with a risk of a skin injury may be acquired, by deriving one or more parameters from the actual physiological signal data and/or by obtaining one or more parameters from external sources, such as an external temperature sensor or an external acceleration transducer.

FIG. 6 illustrates one embodiment of a low power pulse oximeter system provided with site timing functionality. The system comprises a smart sensor 600 attachable to a subject and a central unit 607 adapted to communicate with the smart sensor. The smart sensor normally includes two or more light emitting elements, such as LEDs, and at least one photo detector 603. It is assumed here that the smart sensor includes two LEDs 602, each emitting light at a dedicated wavelength. The wavelength values widely used are 660 nm (red) and 940 nm (infrared). The light emitted by the LEDs and propagated through (or reflected from) the tissue, such as finger 608, is received by the photo detector 603 which converts the optical signal received at each wavelength into an electrical signal.

The smart sensor further comprises a control unit 601, such as a microcontroller, that controls the LEDs through a LED control interface 604, and an A/D converter 605 that converts the electrical signal obtained from the photo detector into digitized format. The control unit receives the (photo) plethysmographic signal data from the A/D converter, and there may also be an amplifier between the photo detector and the control unit. The control unit is connected to a radio frequency interface 606 for transmitting the plethysmographic signal data to the central unit 607 and for receiving data from the central unit. Thus, a two-way communication link 609 exists between the smart sensor and the central unit.

For controlling the LEDs, the control unit 601 is provided with a LED control algorithm 610 configured to control, when executed by the control unit, the LEDs 602 through the LED control interface 604. The central unit 607 is provided with a LED control algorithm 611 that co-operates with algorithm 610, and with an SpO2 calculation algorithm 612. The algorithms 610 and 611 handle the synchronization of the LED operation with the plethysmographic waveforms and the SpO2 calculation algorithm 612 is configured to determine the SpO2 values.

In various embodiments of the pulse oximeter system of FIG. 6, plethysmographic signal data may be collected only during certain waveform sections. Therefore, during the recording of the data the LEDs may be switched on during the said sections only. In some embodiments of the system, however, the LEDs may also be used to synchronize the data collection with the said sections. The functionalities of the LED control algorithm 610 depend on the synchronization mechanism and on how the synchronization functionalities are divided between the sensor and the central unit, i.e. between algorithms 610 and 611.

In practice, the smart sensor of FIG.6 may be divided into two different units; a sensor 613 comprising the optical components of a conventional sensor, i.e. LEDs 602 and photo detector 603, and a base unit comprising the non-optical components of the smart sensor 600. The sensor 613, which is attachable to the subject, may be connected through a short cable to the base unit. In this way, the smart sensor may be divided between a disposable unit, i.e. sensor 613, and a unit with longer durability, i.e. the base unit.

The site timing algorithm 614 may reside either in the control unit 601 or in the central unit 607, but the functionality may also be distributed between the two units. If the algorithm resides in the control unit 601, the base unit (or the disposable unit) may be provided with a visual indicator 615, such as LED, that indicates the need to change the sensor to another measurement site in response to a timer expiration. The sensor/base unit may also be provided with a start button 616, the pressing of which is regarded as a start event.

In the above embodiments, the notification generated upon timer expiration is a user notification given to the user. However, the notification may also be a machine-interpretable notification based on which the apparatus automatically changes the sensor site, i.e. the active sensor from which the physiological signal is measured. This embodiment may be used especially if the cooling down of the sensor, which occurs as a result of the sensor deactivation, is a major factor that prevents a skin injury from developing. Although machine-interpretable notifications may be invisible to the user, the apparatus may indicate the sensor that is active at any given time. Below, a notification indicative of a need to change sensor placement, i.e. the site of the active sensor, may thus refer to a user notification recommending that the sensor placement be changed or to a machine-interpretable notification based on which the apparatus is adapted to change the active sensor.

In terms of sensor placement control, the functionalities of the control and processing unit (or the control unit of the sensor) may be divided into the units shown in FIG. 7. An event detection unit 71 is configured to monitor the occurrence of at least one predetermined event and to indicate the detection of a particular event to a timer control unit 72. Generally, the events monitored may be divided between start events and control events and one or more start events may start the same timer. However, in a simple embodiment the control events may be omitted and only one predetermined start event, such as the start of the measurement or the pressing of a start button, may be used. In a further simple embodiment, only one control event, a user-originated timer control, may be used in addition to a single predetermined start event. The timer control unit is configured to start a timer 73 in response to a start event and control a timer in response to a control event. The control may include control of the settings 74 of one or more timers in response to user input through user interface 104. That is, a specific user action may be regarded as a predetermined control event. An indication unit 75 is configured to produce the notifications and timer state information based on the data obtained from the timer control unit. The notifications may be supplied to the user interface or to the control unit, depending on whether user notifications or machine-interpretable notifications are used. As obvious from the embodiment of FIG. 5, the event detection unit may further be configured to monitor a physiological signal, such as a plethysmographic signal, derive a parameter from the physiological signal, and detect a predetermined event when the parameter fulfills predetermined criteria, such as crossing of a predetermined threshold value. The parameter may be any parameter that correlates with the skin injury risk, such as PI parameter or motion parameter, and several different parameters may be determined. The parameter(s) may also be obtained from a separate measurement device, such as a motion detector unit (acceleration transducer) or a temperature sensor.

A physiological monitoring apparatus, such as a pulse oximeter, may also be upgraded to a device provided with the site timing functionality. Such an upgrade may be implemented by delivering to the apparatus a software module that enables the device to detect at least one predetermined event, start at least one timer in response to the detection of a predetermined start event, and to generate a notification to the user in response to a timer expiration. The software module may be delivered, for example, on a data carrier, such as a CD or a memory card, or through a telecommunications network. The software module may thus include the site timing algorithm shown in FIGs. 1, 3, and 6 and it may also include any one or more of the above features.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural or operational elements that do not differ from the literal language of the claims, or if they have structural or operational elements with insubstantial differences from the literal language of the claims.

Various aspects and embodiments of the present invention are defined by the following numbered clauses:
1. A method for controlling sensor placement time in a physiological measurement apparatus, the method comprising
   - monitoring for occurrence of at least one predetermined event, wherein the at least one predetermined event comprises at least one predetermined start event;
   - upon detection of any of the at least one predetermined start event, starting a respective timer, thereby to start at least one timer, wherein each timer is provided with dedicated timer settings; and
   - upon expiration of any of the at least one timer, generating a notification indicative of a need to change sensor placement.
2. The method according to clause 1, wherein the monitoring includes monitoring for the occurrence of the at least one predetermined event, wherein the at least one predetermined event further comprises at least one predetermined control event.
3. The method according to clause 1 or clause 2, further comprising storing timer settings for the at least one timer, each of the at least one timer being associated with at least one of the at least one predetermined start event.
4. The method according to any preceding clause, wherein the monitoring comprises acquiring a parameter that correlates with a risk of a skin injury and detecting a predetermined control event when the parameter fulfills at least one predetermined criterion, wherein the predetermined control event belongs to the at least one predetermined control event.
5. The method according to any preceding clause, further comprising updating a respective timer in response to a predetermined control event that belongs to the at least one predetermined control event, wherein the updating comprises storing updated timer settings, and wherein the updated timer settings change operation of respective timer if the respective timer is currently running.
6. The method according to any preceding clause, wherein the monitoring comprises monitoring for occurrence of a single predetermined event, thereby to start a single timer.
7. The method according to any preceding clause, wherein the generating comprises generating the notification, in which the notification is a user notification recommending that the sensor placement be changed.
8. A physiological measurement apparatus comprising
   - a sensor attachable to a subject so that the sensor comes into contact with the subject's skin;
   - an event detection unit configured to monitor for occurrence of at least one predetermined event, wherein the at least one predetermined event comprises at least one predetermined start event;
   - a timer control unit configured to start a respective timer in response to detection of any of the at least one a predetermined start event, thereby to start at least one timer, wherein each timer is provided with dedicated timer settings; and
   - an indication unit configured to generate, in response to expiration of any of the at least one timer, a notification indicative of a need to change sensor placement.
9. The physiological measurement apparatus according to any preceding clause, wherein the at least one predetermined event further comprises at least one predetermined control event.
10. The physiological measurement apparatus according to any preceding clause, wherein the timer control unit is further configured to store timer settings for the at least one timer, each of the at least one timer being associated with at least one of the at least one predetermined start event.
11. The physiological measurement apparatus according to any preceding clause, wherein the event detection unit is further configured to acquire a parameter that correlates with a risk of a skin injury and detect a predetermined control event when the parameter fulfills at least one predetermined criterion, wherein the predetermined control event belongs to the at least one predetermined control event.
12. The physiological measurement apparatus according to any preceding clause, wherein the timer control unit is further configured to update a respective timer in response to a predetermined control event that belongs to the at least one predetermined control event, thereby to store updated timer settings, and wherein the updated timer settings change operation of respective timer if the respective timer is currently running.
13. The physiological measurement apparatus according to any preceding clause, wherein the event detection unit is configured to monitor for occurrence of a single predetermined event, thereby to start a single timer.
14. The physiological measurement apparatus according to any preceding clause, wherein the parameter belongs to a group of parameters including perfusion index, a motion parameter indicative of motion of the sensor, and a temperature parameter indicative of temperature of skin surface.
15. The physiological measurement apparatus according to any preceding clause, wherein the notification is a user notification recommending that the sensor placement be changed.
16. A sensor for a physiological measurement apparatus, the sensor being attachable to a subject so that the sensor comes into contact with the subject's skin, the sensor comprising
   - an event detection unit configured to monitor for occurrence of at least one predetermined event, wherein the at least one predetermined event comprises at least one predetermined start event;
   - a timer control unit configured to start a respective timer in response to detection of any of the at least one a predetermined start event, thereby to start at least one timer, wherein each timer is provided with dedicated timer settings; and
   - an indication unit configured to generate, in response to expiration of any of the at least one timer, a notification indicative of a need to change sensor placement.
17. The sensor according to any preceding clause, wherein the event detection unit is configured to monitor for occurrence of a single predetermined event, thereby to start a single timer.
18. The sensor according to any preceding clause, wherein the single predetermined event is a user action.
19. The sensor according to any preceding clause, wherein the user indication unit comprises a visual indicator responsive to the expiration.
20. A computer program product for a physiological monitoring apparatus, the computer program product comprising
   - a first program product portion configured to monitor for occurrence of at least one predetermined event, wherein the at least one predetermined event comprises at least one predetermined start event;
   - a second program product portion configured to start a timer in response to detection of any of the at least one a predetermined start event, thereby to start at least one timer, wherein each timer is provided with dedicated timer settings; and
   - a third program product portion configured to generate, in response to expiration of any of the at least one timer, a notification indicative of a need to change sensor placement.

## Claims

1. A method for controlling sensor placement time in a physiological measurement apparatus, the method comprising
- monitoring (201, 202; 415) for occurrence of at least one predetermined event, wherein the at least one predetermined event comprises at least one predetermined start event;
- upon detection of any of the at least one predetermined start event, starting (203; 416) a respective timer, thereby to start at least one timer (73), wherein each timer is provided with dedicated timer settings (74); and
- upon expiration of any of the at least one timer, generating (205; 420) a notification indicative of a need to change sensor placement.

2. The method according to claim 1, wherein the monitoring includes monitoring for the occurrence of the at least one predetermined event, wherein the at least one predetermined event further comprises at least one predetermined control event.

3. The method according to claim 1 or claim 2, further comprising storing timer settings (74) for the at least one timer, each of the at least one timer being associated with at least one of the at least one predetermined start event.

4. A physiological measurement apparatus comprising
- a sensor (120; 600, 613) attachable to a subject so that the sensor comes into contact with the subject's skin;
- an event detection unit (71, 101; 71, 601) configured to monitor for occurrence of at least one predetermined event, wherein the at least one predetermined event comprises at least one predetermined start event;
- a timer control unit (72, 101; 72, 601) configured to start a respective timer in response to detection of any of the at least one a predetermined start event, thereby to start at least one timer (73), wherein each timer is provided with dedicated timer settings (74); and
- an indication unit (75, 101; 75, 601) configured to generate, in response to expiration of any of the at least one timer, a notification indicative of a need to change sensor placement.

5. The physiological measurement apparatus according to claim 4, wherein the at least one predetermined event further comprises at least one predetermined control event.

6. The physiological measurement apparatus according to claim 4 or claim 5, wherein the timer control unit (72, 101; 72, 601) is further configured to store timer settings (74) for the at least one timer (73), each of the at least one timer being associated with at least one of the at least one predetermined start event.

7. The physiological measurement apparatus according to any of claims 4 to 6, wherein the event detection unit (71, 101; 71, 601) is further configured to acquire a parameter that correlates with a risk of a skin injury and detect a predetermined control event when the parameter fulfills at least one predetermined criterion, wherein the predetermined control event belongs to the at least one predetermined control event.

8. The physiological measurement apparatus according to any of claims 4 to 7, wherein the timer control unit is further configured to update a respective timer in response to a predetermined control event that belongs to the at least one predetermined control event, thereby to store updated timer settings, and wherein the updated timer settings change operation of respective timer if the respective timer is currently running.

9. The physiological measurement apparatus according to any of claims 4 to 8, wherein the event detection unit is configured to monitor for occurrence of a single predetermined event, thereby to start a single timer.

10. The physiological measurement apparatus according to any of claims 4 to 9, wherein the parameter belongs to a group of parameters including passion index, a motion parameter indicative of motion of the sensor, and a temperature parameter indicative of temperature of skin surface.

11. The physiological measurement apparatus according to any of claims 4 to 10, wherein the notification is a user notification recommending that the sensor placement be changed.

12. A sensor for a physiological measurement apparatus, the sensor being attachable to a subject so that the sensor comes into contact with the subject's skin, the sensor comprising
- an event detection unit (71, 101; 71, 601) configured to monitor for occurrence of at least one predetermined event, wherein the at least one predetermined event comprises at least one predetermined start event;
- a timer control unit (72, 101; 72, 601) configured to start a respective timer in response to detection of any of the at least one a predetermined start event, thereby to start at least one timer (73), wherein each timer is provided with dedicated timer settings (74); and
- an indication unit (75, 101; 75, 601) configured to generate, in response to expiration of any of the at least one timer, a notification indicative of a need to change sensor placement.

13. The sensor according to claim 12, wherein the event detection unit is configured to monitor for occurrence of a single predetermined event, thereby to start a single timer, in which the single predetermined event is a user action.

14. The sensor according to claim 12 or claim 13, wherein the user indication unit comprises a visual indicator responsive to the expiration.

15. A computer program product for a physiological monitoring apparatus, the computer program product comprising
- a first program product portion configured to monitor for occurrence of at least one predetermined event, wherein the at least one predetermined event comprises at least one predetermined start event;
- a second program product portion configured to start a timer in response to detection of any of the at least one a predetermined start event, thereby to start at least one timer (73), wherein each timer is provided with dedicated timer settings (74); and
- a third program product portion configured to generate, in response to expiration of any of the at least one timer, a notification indicative of a need to change sensor placement.
